Europäisches Patentamt

European Patent Office

Office européen des brevets

(19)

(11) Publication number: **0 467 416 A1**

## EUROPEAN PATENT APPLICATION

(12)

(21) Application number: **91115720.4**

(22) Date of filing: **03.09.84**

(51) Int. Cl.⁵: **A61K 47/48**, A61K 37/66, A61K 45/06, A61K 39/395

---

This application was filed on 17 - 09 - 1991 as a divisional application to the application mentioned under INID code 60.

(30) Priority: **01.09.83 GB 8323428**

(43) Date of publication of application:
**22.01.92 Bulletin 92/04**

(60) Publication number of the earlier application in accordance with Art.76 EPC: **0 136 835**

(84) Designated Contracting States:
**AT BE CH DE FR GB IT LI LU NL SE**

(71) Applicant: **HYBRITECH INCORPORATED**
**11085 Torreyana Road**
**San Diego CA 92121(US)**

(72) Inventor: **Frincke, James M.**
**339 Shoemaker Lane**
**Solana Beach, California 92075(US)**
Inventor: **Unger, Barbara W.**
**13890 Amber Sky Lane**
**San Diego, California 92121(US)**
Inventor: **Burnett, Karen G.**
**3814 Martha Street**
**San Diego, California 92117(US)**
Inventor: **Hersh, Evan Myron**
**9 Falling Leaf**
**Houston, Texas 77024(US)**
Inventor: **Rosenblum, Michael Gordon**
**8810 No. Rylander Circle**
**Houston, Texas 77071(US)**
Inventor: **Gutterman, Jordan Udell**
**5943 Yarwell Drive**
**Houston, Texas 77035(US)**

(74) Representative: **Cresswell, Thomas Anthony**
**J.A. Kemp & Co. 14 South Square Gray's Inn**
**London WC1R 5LX(GB)**

---

(54) **Antibody compositions of therapeutic agents having an extended serum half-life.**

(57) A therapeutic agent e.g. alpha-interferon, complexes with an antibody without impairing the activity. The antibody can be a monoclonal antibody. The serum half-life of the therapeutic agent administered as the complex is substantially increased when compared to that of the agent administered alone.

## Field of the Invention

This invention relates to therapeutically active agents and the treatment of disease therewith. In another aspect, it relates to antibody complexes of a therapeutically active agent. In a more specific aspect, it relates to complexes of a monoclonal antibody and a therapeutically active agent and their use in the treatment of disease.

## Background

It is almost a trite observation to note that the use of a broad spectrum of drugs to treat human and other mammalian disease is routine medical and veterinary practice. Therapeutically active agents, however, often suffer from a number of shortcomings which limit and complicate their use. A particular problem is that, after administration to the patient, a drug may be so rapidly cleared from the body by metabolic or other pathways or otherwise biologically inactivated so that only a relatively small percentage of the drug administered actually has a therapeutic effect. To compensate for this problem, it is common practice to increase the dosage of the drug and/or to prolong its period of administration and/or to shorten the interval between doses so that the therapeutically effective concentration of the drug in the patient is maintained for a period sufficient to achieve the desired result.

These procedures are useful but have their own limitations. Increasing the dosage may be limited, for example, in the case of intramuscular administration, by the bolus which can be tolerated. Many drugs have toxic side effects which may limit the dosage duration or interval which can be safely used. In some cases, promising drugs cannot be used because side reactions are so severe that an effective therapeutic dose cannot be safely administered. The need to administer multiple small doses of a drug or to use continuous infusion techniques increases the cost of treatment and the burden on hospital personnel, and, of course, adds to the patient's discomfort.

Accordingly, there exists a need for means by which the therapeutically active concentration of a drug, after administration, is maintained for a longer time.

## Summary of the Invention

It is the normal and expected function of antibodies to complex with foreign substances to more rapidly clear them from the body. We, however, have unexpectedly found that the serum or plasma half-life of a therapeutically active agent can be extended by forming a complex of the agent with a selected antibody, preferably a monoclonal antibody, which binds to the agent at a site which does not substantially impair its therapeutic activity and which extends the serum half-life of the agent. Thus, as used herein, the term "antibody" means a monoclonal antibody or polyclonal antibodies unless otherwise specified or required by the context. According to our invention, the complex of the therapeutically active agent and the antibody may be formed in vitro and then administered. Alternatively, the agent and antibody may be administered at the same time. In yet another alternative, the antibody may be administered first, and after an interval during which its distribution in the patient approaches equilibrium, the therapeutically active agent can be administered.

By selecting the proper antibody for forming the antibody: drug complex, the serum half-life and, thus, the effective concentration of the therapeutically active agent, can be maintained in vivo for a longer interval. While monoclonal antibodies are preferred for use in the invention, it is also within the scope of the invention to use polyclonal antibodies against the therapeutically active agent which complex with the therapeutically active agent without materially impairing its therapeutic activity.

Accordingly, it is an object of the present invention to provide means by which the serum half-life of a therapeutically active agent is extended.

Another object of the invention is to provide compositions which increase the effective lifetime of a therapeutic agent in vivo after administration to a patient.

## Detailed Description of the Invention

As indicated above, the present invention, in one embodiment, is a complex between a therapeutically active agent with a monoclonal antibody selected to bind the therapeutic agent at a site which does not materially impair its therapeutic activity but which forms a complex with the agent to confer upon the agent a serum half-life longer than that of the therapeutic agent alone and approaching the serum half-life of the antibody. Alternatively, the invention comprises a similar complex of therapeutic agent with polyclonal antibodies selected to bind the antibody without materially impairing its therapeutic activity and which form a complex having an extended serum half-life.

In another embodiment, the invention is a process involving the administration to a host of a complex comprising the therapeutic agent and either a monoclonal antibody or polyclonal antibodies having the properties noted above. The process of the present invention also includes either simultaneous administration of the therapeutics agent and a suitable antibody preparation or an initial administration of the antibody preparation followed by administration of the therapeutic agent after the antibody has had an opportunity to distribute itself throughout the host.

The therapeutic agents useful in the invention are those which are or can be made immunogenic, i.e., those for which an immune response can be obtained either directly or, in the case of a hapten, by binding the agent to a molecule which is immunogenic. Monoclonal antibodies against the therapeutic agent can be obtained by methods which are now well known to the art and which need not be described in detail. These methods generally involve immmunization of a mouse or other animal species, usually mammalian or avian, with the immunogen. Human lymphoid cells may also be obtained after immunization (natural or induced) or may be sensitized in vitro. After an immune response is generated, spleen cells of the immunized mouse or other immune lymphoid cells are fused with cells of an established lymphoid tumor line using known techniques to form hybridomas which produce monoclonal antibodies. Clones of hybridomas are screened to select those which are producing monoclonal antibodies that are specific for the antigen of choice, in this case the therapeutic agent. Monoclonal antibodies having the desired specificity are further screened to select those that form an antibody:agent complex in which the agent retains all, or substantially all, of its therapeutic activity. These complexes are further screened to select those which have an extended serum half-life. In certain circumstances, it can be beneficial to use a mixture of two or more monoclonal antibodies. In some circumstances it may also be desirable to use a stoichiometric excess of antibody.

Polyclonal antibodies useful in the invention are obtained by well known techniques as well. These include stimulating an immune response against the therapeutic agent, or fragment thereof, in a suitable animal host such as a rabbit or other mammal. Chickens and other avian species can also be used. Serum taken from the host is subjected to affinity purification to isolate polyclonal antibodies against the therapeutic agent. These antibodies are subsequently fractionated, if necessary, to isolate a subpopulation which complexes with the therapeutic agent without materially impairing its desirable activity.

Particularly preferred for use in the invention are human antibodies against the therapeutic agent produced by hybridomas which, for example, are the product of fusion of a human B-lymphocyte with an established mammalian lymphoid line, e.g., a human or mouse myeloma line.

As used herein, the term antibody includes fragments thereof such as Fab, Fab', and Fab'2 or mixtures thereof and including mixtures with whole antibody. Such fractions may be less immunogenic in some patients and may also better allow better penetration of the agent to the target site.

In certain applications, the monoclonal antibody is preferably a hybrid antibody having a dual specificity, one against the therapeutically active agent and the other against another antigen, for example, an antigen associated with the disease which it is desired to treat with the agent. Among these may be mentioned tumor associated antigens such as carcinoembryonic antigen (CEA), prostatic acid phosphatase (PAP), ferritin and prostate specific antigen (PSA). In such cases, the other specificity could be selected to bind with an agent which has anti-tumor activity. For example, the second specificity could be against a toxin such as ricin or an interferon. Processes for obtaining such hybrids are disclosed in the pending patent application of J. Martinis et al., Serial No. 367,784, filed April 12, 1983, assigned to Hybritech Inc., European Patent Appln. 0-105-360 the disclosure of which is incorporated by reference.

Among the therapeutic agents which are useful in the invention may be mentioned drugs such as adriamycin, vincristine, genomycin mitomycin C, and prostacyline; toxins such as abrin and ricin; and biological proteins such as the interferons (alpha, beta and gamma), the interleukine, hormones such as insulin, plasminogen activators such as urokinase, streptokinase and tissue plasminogen activator, growth factors such as nerve growth factors, and platelet activating factor. Particularly useful are complexes of a monoclonal antibody and one of the interferons, for example, alpha-interferon. As used herein, the term "interferon" is used to include those agents having the characteristics attributed to interferons as described in Interferon: An Overview, Ion Gresser, Ed., 4 (1982), p. 4, which is incorporated herein by reference. It is also used to mean an interferon as it occurs naturally or a synthetic modification thereof. In particular, it includes an interferon made by recombinant DNA technology which is identical to a naturally occurring interferon or which differs therefrom by one or more of the following:

1. a difference in amino acid sequence;
2. a difference in chain folding;
3. a difference in carbohydrate substitution.

The utility of the present invention is shown by the experiments described below with alpha-interferon. In that regard, alpha-interferon, a multi-species interferon, has been shown to have a therapeutic effect in the treatment of certain malignant tumors including breast cancer, multiple myeloma and malignant lymphoma. However, it has been shown to rapidly clear from the plasma of man and animals during clinical trials. This has been compensated for by giving a high dose intra-muscularly. However, the maximum dose is limited because of high-dose toxic side effects. Also, the high doses used are very expensive and may elicit an immune response in a substantial number of patients treated.

The following Example is given to illustrate the invention.

1. Preparation of anti-alpha-interferon monoclonal antibodies:

Balb/c mice were immunized with partially purified leukocyte interferon. Spleen cells from immunized mice were fused with a myeloma line (either the NS-1 or SP2/0 lines) to produce hybridomas. The hybridomas were screened to select those reactive with $^{125}$I-labeled interferon in a radioimmunoassay wherein the immune complexes were removed by horse anti-mouse IgG bound to sepharose beads. Interferon used in immunization and screening were from the same source. Antibodies were selected for positive reactivity with interferon. Hybridomas producing the selected antibodies were cloned by limiting dilution to ensure homogeneity of the cell population.

2. Testing for Reactivity of an Antibody:Interferon Immune Complex in the Anti-Viral Protection Assay

Approximately 40 anti-alpha interferon monoclonal antibodies were employed to make interferon:antibody immune complexes which were tested for retention of anti-viral activity using the standard method described, for example, in Rubinstein, et al., J. Virology, 37, 755 (1981). The first step in this procedure was formation of the immune complex by the addition of ascitic fluid to the anti-viral protection assay mixture which was monitored for inhibition of interferon activity. Ten of the forty antibodies were selected for further investigation because they did not inhibit the viral protection properties of the interferon in this assay. These antibodies were then further concentrated with sodium sulfate and re-tested. In each case, non-inhibition of anti-viral activity as verified. To demonstrate whether complexes of interferon with these antibodies were actually formed, the reaction mixtures were adsorbed with solid phase sepharose bound sheep anti-mouse IgG to remove the antibody and complexed interferon. The supernatant from the sepharose adsorptions were then tested in the standard antiviral protection assay. In the case of one particular antibody, designated IFG 252.2 by us, the antiviral protection was almost completely removed from the supernatant during the adsorption. Controls were performed to ensure this phenomena was not due to non-specific absorption during the sepharose adsorption step. These data demonstrate that this antibody binds efficiently and avidly to interferon without inhibiting its antiviral activity.

Another known biological property of alpha interferon is its inhibition of cellular proliferation. In an assay system using DAUDI cells, retention of anti-proliferative activity was demonstrated for alpha interferon in the presence of the IFG 252.2 antibody. These data demonstrate that IFG 252.2 also binds alpha-interferon without affecting its anti-proliferative activity.

3. Administration of Alpha-Interferon:IFG 252.2 Complex to Laboratory Rats

A Fisher rat (250-260 g) was lightly anesthetized with sodium thiopental. A plastic canula was then surgically inserted into the femoral artery of the other leg. A bolus dose of alpha-interferon (Clone A of Goeddel et al., Nature, 290, 20-26 (1981), 7600 units total in 0.5 ml phosphate buffered saline) was administered over 2 seconds into the venous catheter. Blood samples (0.5 ml) were withdrawn at various times from the arterial catheter. After each blood withdrawal, 0.5 ml of PBS were injected via the venous catheter. The samples were centrifuged, the plasma decanted and analyzed for interferon anti-viral activity by standard methods. In a second rat, the same amount of interferon was preincubated with IFG 252.2 (38 microgram/microliter = 190 micrograms antibody) and then administered through the venous catheter. Blood samples were taken and analyzed in the same way as for the first. The results of those experiments were then plotted and subjected to nonlinear regression analysis.

These results indicate that the activity of alpha-interferon in the rat without added anti-interferon has a two phase disappearance curve. The alpha-phase has a 6.8 minute half-life with a two log reduction of interferon activity in the plasma at 30 minutes. The volume of distribution is 20.8 ml. At 30 minutes a beta component to the plasma disappearance curve is identified with a 30 minute half-life. At two hours essentially all of the interferon activity has been lost from the plasma. The area under the curve was 7047 u/ml x min. In contrast, when the IFG 252.2 antibody is utilized to extend the half-life, a single phase disappearance of activity from plasma is observed. The half-life of this activity loss is 84 minutes. Twelve times longer than that observed for alpha-interferon itself, with a volume of distribution of 19.2 ml, essentially equivalent to that observed for alpha-interferon without added antibody. The area under the curve was 50,000 u/ml x min, seven (7) times that for the free interferon.

The foregoing experiments demonstrate that, by proper selection of an antibody, the serum half-life of a therapeutically active agent can be usefully extended without signficant impairment of therapeutic activity.

Those skilled in the art will recognize that the invention, therefore, has application in veterinary medicine and for human health care. In that connection, it is within the scope of the invention to combine the therapeutic agent and/or the antibody or the antibody complex with the agent with other components such as a suitable vehicle. The foregoing description of the invention is exemplary only and modifications thereof may be made without departure from the scope of the invention which is to be limited only by the appended claims.

## Claims

1. A composition comprising a complex of a therapeutically active agent and an antibody selected to bind said agent at a site which does not substantially impair its therapeutic activity and which extends the serum half-life of the therapeutically active agent.

2. A composition according to claim 1 wherein the antibody is a monoclonal antibody.

3. A composition according to claim 1 wherein the antibody comprises a population of polyclonal antibodies.

4. A composition according to claim 1 wherein the antibody is a hybrid monoclonal antibody having a dual specificity one of which is against the therapeutically active agent and the other against a disease associated antigen.

5. A composition according to claim 4 wherein one specificity of the hybrid antibody is directed against a tumour associated antigen and the other against an agent having anti-tumour activity.

6. A composition according to claim 5 wherein the tumour associated antigen is CEA, PAP, PSA or ferritin.

7. A composition according to claim 5 or 6 wherein the second specificity is directed against an interferon.

8. A composition according to any one of the preceding claims wherein the antibody comprises an antibody fragment which is Fab, Fab' or Fab'2.

9. A composition according to any one of the preceding claims, wherein the therapeutically active agent is a drug, toxin or biological protein.

10. A composition according to any one of the preceding claims wherein the therapeutically active agent is an interferon.

11. A composition according to claim 10 wherein the interferon is alpha, beta or gamma interferon.

12. A composition according to any one of the preceding claims further comprising a pharmaceutical vehicle.

13. A composition according to any one of the preceding claims for use in a process for treatment of the human or animal body by surgery or therapy or in a diagnostic method practised on the human or animal body.

14. A composition according to claim 13 wherein the process is one in which the antibody and agent are combined in vitro.

15. A composition according to claim 13 wherein the process is one in which the antibody and agent are separately administered to the human or animal body.

16. A composition according to claim 15 wherein the process is one in which the antibody is allowed to distribute itself throughout the human or animal body prior to administration of the agent.

## Claims for the following Contracting State: AT

1. A process for the production of a complex of a therapeutically active agent and an antibody selected to bind said agent at a site which does not substantially impair its therapeutic activity and which extends the serum half-life of the therapeutically active agent which comprises combining the antibody and agent in vitro.

2. A process for the production of a complex of a therapeutically active agent and an antibody selected to bind said agent at a site which does not substantially impair its therapeutic activity and which extends the serum half-life of the therapeutically active agent which comprises combining the antibody and agent in a host in vivo.

3. A process according to claim 2 wherein the antibody is distributed throughout the host prior to the introduction of the agent.

4. A process according to any one of the preceding claims wherein the antibody is a monoclonal antibody.

5. A process according to any one of claims 1 to 3 wherein the antibody comprises a population of polyclonal antibodies.

6. A process according to any one of claims 1 to 3 wherein the antibody is a hybrid monoclonal antibody having a dual specificity one of which is against the therapeutically active agent and the other against a disease associated antigen.

7. A process according to claim 6 wherein one specificity of the hybrid antibody is directed against a tumour associated antigen and the other against an agent having anti-tumour activity.

8. A process according to claim 7 wherein the tumour associated antigen is CEA, PAP, PSA or ferritin.

9. A process according to claim 7 or 8 wherein the second specificity is directed against an interferon.

10. A process according to any one of the preceding claims wherein the antibody comprises an antibody fragment which is Fab, Fab' or Fab'2.

11. A process according to any one of the preceding claims wherein the therapeutically active agent is a drug, toxin or biological protein.

12. A process according to any one of the preceding claims wherein the therapeutically active agent is an interferon.

13. A process according to claim 12 wherein the interferon is alpha, beta or gamma interferon.

14. A process according to any one of the preceding claims further comprising a pharmaceutical vehicle.

European
Patent Office

EUROPEAN SEARCH
REPORT

Application Number

EP 91 11 5720

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 068 763 (THE BOARD OF REGENTS UNIVERSITY OF TEXAS SYSTEM)<br>* Page 1, paragraph 1; page 4, paragraph 1; page 5, paragraph 3; page 1, paragraph 3; page 12, paragraph 2; claims 1-4 *<br>– – – | 1-16 | A 61 K 47/48<br>A 61 K 37/66<br>A 61 K 45/06<br>A 61 K 39/395 |
| X,D,P | WO-A-8 303 679 (HYBRITECH INC.)<br>* Page 1, lines 2,3; page 5, lines 16-26; page 6, lines 18-28; page 7, lines 5-7,14-16; page 32, lines 19-35; claims 1,6,7,18-20,25-28,58,61-71 *<br>– – – | 1-16 | |
| E | EP-A-0 137 234 (THE WELLCOME FOUNDATION LTD)<br>* Page 2, lines 12-14,23-27,30-35; page 3, lines 4-30; page 4, lines 4-13,23-28; claims 1,5,11-13 *<br>– – – | 1-3,8-16 | |
| A | US-A-4 195 017 (S. BOGOCH)<br>* Column 2, lines 57-62,65-67; column 3, lines 1-4,12-24,35-42,47-51; column 5, lines 34-41; claims 1-3,5,6 *<br>– – – | 1,3,8,9, 12-16 | |
| A | EP-A-0 044 167 (THE REGENTS OF THE UNIVERSITY OF CALIFORNIA)<br>– – – – – | | |

| | | | TECHNICAL FIELDS SEARCHED (Int. Cl.5) |
|---|---|---|---|
| | | | A 61 K |

The present search report has been drawn up for all claims

| Place of search | Date of completion of search | Examiner |
|---|---|---|
| The Hague | 11 November 91 | RYCKEBOSCH A.O.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document